# EUROPEAN PATENT APPLICATION

(11) **EP 4 782 815 A1**
(43) Date of publication of application: **29.07.2026**
(21) Application number: 25154008.4
(22) Date of filing: 27.01.2025
(51) Int. Cl.: G01N 15/149, C12M 1/00, B01L 9/00, C12M 1/36, G01N 35/10, B01L 1/00, C12M 3/06, G01N 15/00, G01N 35/00

(54) **SORTING DEVICE AND CORRESPONDING SORTING METHOD**

(71) Applicant: CSEM Centre Suisse d'Electronique et de Microtechnique SA - Recherche et Développement, 2002 Neuchâtel (CH)
(72) Inventor: BURCH, Thomas, 6062 Wilen (CH)
(74) Representative: e-Patent SA

(57) **Abstract**

A sorting device (1) comprising a picking station (4) and an identification and localization system, adapted to allow selective picking up of an biological entity from a source vessel (2) containing a sample including a plurality of biological entities, to selectively dispense the selected biological entity into a destination vessel (6), the picking station (4) including a vessel support (30) for bearing a source vessel (2), mounted in a movable manner, as well as a control unit and a driving organ adapted to control the movements of the vessel support (30), so as to drive the latter along looped trajectories with predefined shapes and speed parameters which are adapted to stir the sample and provide relative movements of the plurality of biological entities between each other and with respect to the source vessel (2).

## Description

### Technical Field

The invention is in the field of the handling and sorting of sensitive biological entities, essentially multicellular entities, such as cell aggregates, cell colonies, organoids, spheroids, zebrafish eggs, zebrafish larvae, c. elegans, tissue sections, biopsy tissue, assembloids or others, herein generally named by "biological entities" in the context of the present disclosure.

More specifically, the invention relates to a sorting device comprising a picking station and an identification and localization system, adapted to allow selective picking up of at least one biological entity from a source vessel containing a sample including a plurality of biological entities, to be able to selectively dispense the at least one biological entity into a destination vessel, the sorting device including a frame on which a vessel support for bearing at least one source vessel is mounted in a movable manner, as well as a control unit and a driving organ adapted to control the movements of the vessel support with respect to the frame.

### State of the art

Organoids are three-dimensional structures grown from stem cells that mimic the structure and function of organs. They are typically cultivated in laboratory settings under controlled conditions, allowing researchers to study aspects of organ development, disease progression, and drug responses in a more accurate and ethically acceptable manner compared to traditional animal models or cell cultures. Those biological entities represent a powerful tool in biomedical research with diverse applications spanning from basic science to clinical translation. The size of an organoid can range from very small structures resembling a few hundred micrometers in diameter to larger, more complex structures that can be several millimeters in size.

Depending on factors like cell source, culture conditions, culture techniques among others, biological entities with very heterogeneous properties can result from their maturation in the same culture sample. Many applications, e.g. drug screening, require a selection step, including quality control and individualization (i.e. separation) of the selected biological entities before the biological assays can be run.

There are several commercial solutions for the inspection and picking of individual cells, small-sized multicellular entities and even organoids. See for example, the "Cellcelector flex" by Sartorius: https://www.sartorius.com/en/products/cell-selection-and-retrieval?_gl=1*28qq1t*_up*MQ..&gclid=Cj0KCQjw0MexBhD3ARIsAEI3WHL Zg3NpyAWDNuTQyccNMEdAHXBzw0Th0IV5y6FTW8teK-igBTHTv9YaAgCDEALw_wcB.

Patent EP2955502B1 discloses a similar automated sorting device including the above-mentioned features. More particularly, this sorting device comprises an image acquiring system arranged to identify and locate biological entities of interest. Once a mapping of the sample contained in a source vessel is done, the source vessel is cautiously moved with high precision, along the xy plane, to bring a biological entity to be selectively picked up in a suction position, where it can be picked up.

In general, the handling of such biological entities with sizes larger than 1 mm (up to 5 mm) is difficult because of their tendency to sediment and their fragility to mechanical stress. One known problem is that in some circumstances, especially when working with cultures in smaller wells, the biological entities may lie close to the recipient walls, and it becomes mechanically impossible to reach them with the pipette tip.

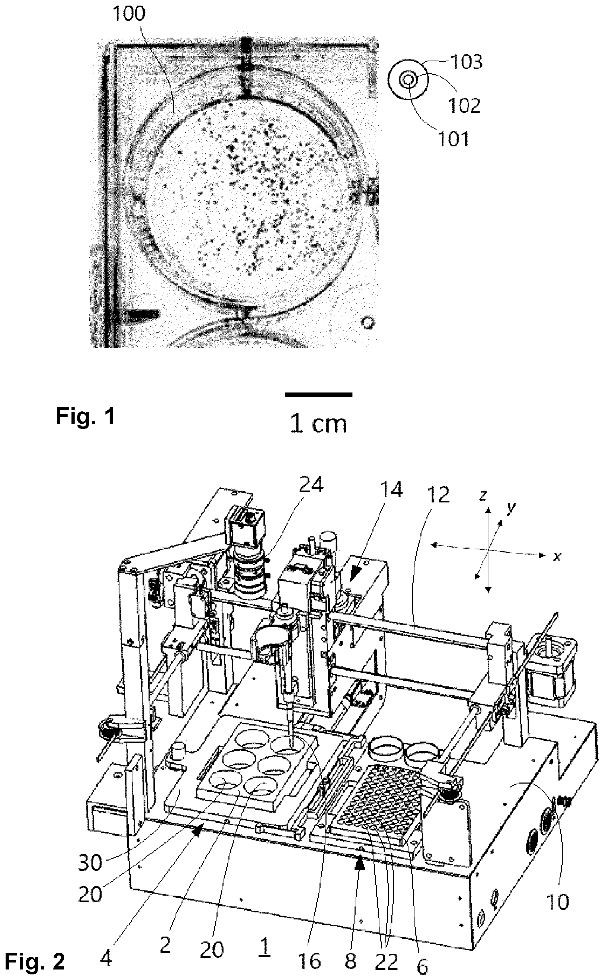

These problematics are illustrated in Fig. 1 which is a photography of a source vessel 100 containing a sample including a plurality of organoids. One can clearly see from Fig. 1 that some of them may be too close to the wall of the source vessel to reach them with a standard pipette tip. The relevant diameters of the pipette tip are also represented at scale in Fig. 1, where 101 is the tip inner diameter, 102 is the outer diameter at the bottom of the tip and 103 is the diameter at the top of the tip.

The automated cell sorting devices of the prior art do not address this problem and, consequently, the need still exists to provide a solution to be able to selectively pick up organoids as other types of sensitive biological entities from a sample with reduced risks of damages caused to one or several of the biological entities included in this sample.

### Disclosure of the invention

An aim of the present invention is to propose a sorting device making it possible to selectively pick up biological entities from a sample with reduced risks of damages that could be caused to one or several of the biological entities included in this sample. Another aim of the present invention is to propose a method taking advantage of the above device for the selective picking up of at least one biological entity from a source vessel containing a sample including a plurality of biological entities, to be able to selectively dispense this picked up biological entity into a destination vessel, in a precise, reliable and non-damaging manner.

These aims are achieved by providing a sorting device as mentioned above, further characterized
by the fact that the control unit and the driving organ are arranged so as to drive the vessel support along successive looped trajectories with respect to the frame, inside a xy plane, in response to a corresponding manual request by a user or to a corresponding automatic request by the identification and localization system, and
by the fact that the control unit and the driving organ are arranged so as to provide the looped trajectories with predefined shapes and speed parameters which are adapted to stir the sample and provide relative movements of the plurality of biological entities between each other and with respect to the source vessel so as to be able to substantially gather the plurality of biological entities inside a central region of the source vessel (2), in the xy plane.

While some sorting devices of the prior art, as mentioned above, may be arranged so as to be able to cautiously move a source vessel from an identification station to a picking station, for instance, after a localization map of the biological entities of interest in a given sample is built up, these devices are generally adapted to move the source vessel by implementation of high resolution linear x, y actuators, in a smooth and slow manner so as to avoid as much as possible any displacement of the biological entities within the sample.

In a different manner, the sorting device of the present invention allows the implementation of a stirring operation of a sample after an analysis of its composition shows a situation which might require to do so because one or several biological entities may lie close to the recipient walls. Indeed, the Applicant noticed that implementation of a stirring in the form of looped trajectories with respect to the frame of the sorting device, with adapted predefined shapes and speed parameters, provide relative movements of the plurality of biological entities between each other and with respect to the source vessel, which may solve the above-mentioned problem.

According to a preferred embodiment of the invention, the control unit and the driving organ may be arranged so as to provide the vessel support with an acceleration having a radial component of at least 0.1 m/s².

In the case of one or several biological entities lying close to the recipient walls, the control unit and the driving organ are advantageously arranged so as to drive the vessel support along successive looped trajectories with a rotation speed of 120 rpm or less, so as to be able to substantially gather the plurality of biological entities inside a central region of the source vessel, in the xy plane.

Generally, the looped trajectories may preferably be substantially circular or elliptical and/or may preferably have a mean characteristic diameter of 2 cm or smaller, preferably 1 cm or smaller.

Generally, the vessel support may advantageously be connected to the frame by at least one elastic blade, preferably at least two elastic blades.

Generally, the driving organ may advantageously comprise a crank arranged in contact with the vessel support to drive the latter along the looped trajectories.

According to a preferred embodiment, the sorting device of the present invention may further be arranged
such that the vessel support has at least one see-through window, and
such that the identification and localization system comprises at least one image acquisition device mounted on the frame and facing the see-through window so as to be able to acquire an image of biological entities included in a sample contained in a transparent source vessel while it is located on the vessel support.

The present invention further relates to a method for the selective picking up of at least one biological entity from a source vessel containing a sample including a plurality of biological entities, this method taking advantage of sorting device having the preceding features to be able to selectively dispense the at least one biological entity into a destination vessel.

More specifically, the method of the present invention advantageously comprises the steps consisting in:
a) providing a sample including a plurality of biological entities in a source vessel and put the latter down on a vessel support of a sorting device as previously mentioned,
b) assessing the distribution in the source vessel of the plurality of biological entities included in the sample, in particular, if the at least one biological entity to be selectively picked up is close to a wall of the source vessel,
c) implementing a stirring operation of the sample, on the basis of a manual request by a user or of an automatic request by the identification and localization system, during which the driving organ drives the vessel support according to successive looped trajectories, inside the xy plane, with predefined shapes and speed parameters which are adapted to stir the sample and provide movements of the plurality of biological entities between each other and with respect to the source vessel so as to be able to substantially gather the plurality of biological entities inside a central region of the source vessel, in the xy plane,
d) acquiring at least one image of the sample to locate the at least one biological entity to be selectively picked up, and
e) selectively picking up the at least one biological entity to be selectively picked up.

According to a preferred embodiment, the vessel support may be driven by the driving organ with an acceleration having a radial component of at least 0.1 m/s² in step c).

In particular, it may be provided that, if it appears in step b) that the at least one biological entity to be selectively picked up is close to a wall of the source vessel, the vessel support is driven, in step c), along successive looped trajectories with a rotation speed of 120 rpm or less, so as to be able to substantially gather the plurality of biological entities inside a central region of the source vessel, in the xy plane.

Generally, the looped trajectories may advantageously be substantially circular or elliptical.

### Brief description of the drawings

Further details of the invention and other advantageous embodiments will appear more clearly upon reading the description below, in connection with the following figures which illustrate:
- Fig. 1: Photography of a standard sample containing organoids illustrating problems which are known in the field of organoid sorting devices;
- Fig. 2: Schematic perspective view of a sorting device according to a preferred embodiment of the present invention;
- Fig. 3: Schematic and simplified lateral view of a sorting device according to a preferred embodiment of the present invention;
- Fig. 4: Schematic front view of a detail of the sorting device of Fig. 2;
- Fig. 5: Schematic and simplified front view of an alternative embodiment of the detail of Fig. 4, and
- Fig. 6: Schematic diagrams illustrating an aspect of a sorting method according to an embodiment the present invention.

### Embodiments of the invention

Fig. 2 is a schematic perspective overall view of a sorting device 1 according to a preferred embodiment of the present invention, in which most of the main components of the sorting device 1 are illustrated.

The sorting device 1 is an inspection and sorting device for sorting sensitive biological entities such as organoids, advantageously by selective picking up of at least one organoid from a source vessel 2, containing a sample including a plurality of organoids and located in a picking station 4, to be able to selectively dispense this organoid into a destination or target vessel 6 located in a delivery station 8. As already mentioned, in the context of the present disclosure, the biological entities of interest are more generally multicellular entities, such as cell aggregates, cell colonies, organoids, spheroids, zebrafish eggs, zebrafish larvae, c. elegans, tissue sections, biopsy tissue, assembloids or others. However, for the sake of simplicity, the remainder of the detailed description will be focusing on organoids in an illustrative and non-limiting manner.

The sorting device 1 comprises a main housing defining a frame 10 and preferably adapted to be installed on a working surface (not illustrated), for instance a table in a laboratory.

The housing is typically provided with different electronic interfaces, at least for power supply, and contains electronic circuits essentially having a well-known architecture in an advantageous manner.

The frame 10 essentially includes the upper side of the housing which delimits here the picking station 4 and the delivery station 8, adjacent to each other in an illustrative non-limiting way.

In Fig. 2, the source vessel 2 is represented in its functional configuration in the picking station 4, while the destination vessel 6 is represented in its functional configuration in the delivery station 8.

The frame 10 further carries a guiding structure 12 for a pipetting unit 14, movable in the xy plane and comprising at least one pipette tip 16 movable in the z direction (either alone, with reference to the pipetting unit 14, or together with it). In a known manner, the pipetting unit 14 can be controlled to move the pipette tip 16 between the picking station 4 and the delivery station 8, where the pipette tip 16 is driven to move along the z direction, to pick up one or several entities/organoids from the source vessel 2 and deliver them in the destination vessel 6.

It should be noted that both source vessel 2 and destination vessel 6 are shown with illustrative and non-limiting shapes. Typically, the source vessel 2 has here several large cylindrical wells 20, each intended to contain a sample (with a liquid or gel) including several organoids as illustrated in Fig. 1, while the destination vessel 6 has more smaller wells 22, each of which is intended to contain one picked up organoid only (sometimes, two or more similar organoids might be delivered in a same small well 22 in a known manner). Of course, the source and destination vessels may have different alternative shapes without going beyond the scope of the invention as defined in the appended claims. For instance, the source vessel may have only one large well 20 and/or the delivery station 8 might be provided with a support arranged for supporting a plurality of individual vessels, each defining one small well 22. More generally, the sample vessels or plates may comprise petri dishes, 6 well-plates, 96 well plates, or any other standard or custom culture recipient.

The sorting device 1 further comprises an identification and localization system allowing to carry out an assessment of the content of a sample contained in a given source vessel 2 as it is installed in its functional configuration in the picking station 4. Many commercially available systems are already known for mapping the content of a sample, generally not only the number of organoids included in the sample and their precise location or distribution within the source vessel, but also the composition of each organoid included in the sample.

For instance, such an identification and localization system may preferably comprise an overview camera 24 arranged above the picking station 4.

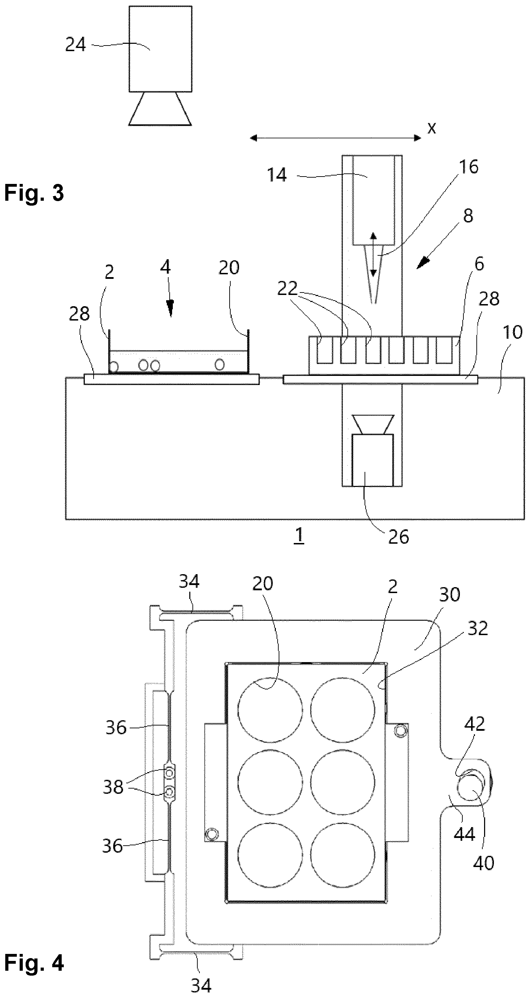

Fig. 3 is a schematic and simplified lateral view of the sorting device 1, from which it appears that, optionally, the identification and localization system might advantageously comprise an additional camera 26 arranged in the opposite direction to that of the overview camera 24.

Either or both of these cameras 24, 26 might be adapted to acquire a magnified image of the content of the sample in order to properly assess the distribution in the source vessel 2 of the plurality of organoids or other biological entities included in the sample. One camera might be arranged so as to acquire images from one of the picking station 4 and the delivery station 8 while the other camera might be arranged to acquire images from the other station. In fact, it also makes sense to assess the content of the destination vessel 6 after an organoid was allegedly delivered, to check if it is indeed the case.

According to a preferred embodiment, the two cameras 24, 26 may have different optical properties (in terms of resolution and/or magnification but also in terms of light spectrum) but one of them might be still with respect to the frame 10, while the other one might be movable between the picking station 4 and the delivery station 8. For instance, the overview camera 24 might be still above the picking station 4 and the additional camera 26 might be mounted on the frame 10 in such a way that it can be moved between the picking station 4 and the delivery station 8, advantageously to follow the pipetting unit 14. Thanks to such an arrangement, cameras 24 and 26 might simultaneously provide images of the picking station 4, at the time of picking up an organoid in a sample, where the corresponding images would provide complementary data, for instance for the purpose of identifying the organoids included in the sample. Then, the additional camera 26 might follow the pipetting unit 14 to the delivery station 8, to be able to check that the delivery of a given organoid in a destination vessel 6 is properly carried out. Advantageously, one of the cameras 24, 26 or an additional part of the identification and localization system may further comprise a microscope system.

Generally, the housing of the sorting device 1 may advantageously comprise one or several see-through windows 28 so that the additional camera 26 may acquire images of the content of the source vessels 2 and of the destination vessels 6 from below, i.e. from the side of the vessels which is opposite to that of the pipetting unit 14. For that purpose, different alternative embodiments may be implemented without any major impact on the present invention. For instance, the housing may comprise portions made of a transparent material (glass or plastics) located in the picking station 4 and/or in the delivery station 8, or the window(s) might even be simply implemented in the form of openings (or at least one could be implemented in the form of an opening and the other might be implemented in the form of a portion made of a transparent material). In general, a transparent part for defining each see-through window 28 is preferred to avoid any risk that part of a sample could be spilled on the additional camera 26.

Thanks to the preceding features, it is possible to take advantage of the fact that most vessels (culture dishes and multi-well plates) are normally transparent, by running the acquisition of images through their bottom. The one skilled in the art will encounter no particular difficulty to adapt the present teaching to his own needs and implement alternative solutions to those which have just been described, as far as the implementation details of the identification and localization system are concerned, as well as the associated features of the housing of the sorting device 1, without going beyond the scope of the invention as defined by the appended claims.

Now referring back to the problematics previously presented in connection with the photography of Fig. 1, in particular, if at least one organoid to be selectively picked up is close to a wall of the source vessel 2 containing the sample, the corresponding solution provided by the present invention will be more precisely explained in connection with Figs. 4, 5 and 6.

Fig. 4 is a detailed view of the support 30 for a source vessel 2, according to a preferred embodiment of the present invention of the sorting device 1, as already illustrated in Fig. 2.

The support 30 has a general shape of a plate, rectangular here in a non-limiting illustrative way, provided with a central aperture 32 having a general shape which is substantially complementary to that of the source vessel 2. Hence, as illustrated, a source vessel 2 can be put down on the support 30 so as to at least partly fit inside the aperture 32, in such a way that the relative position between the source vessel 2 and the support 30 in the xy plane is secured. Specific securing means for the z direction might also be provided.

The support 30 is connected to the housing of the sorting device 1 by a flex mechanism comprising flex structures 34, 36 in the form of blades which can elastically bend, allowing the motion of the support 30 along both the x and the y directions. The flex mechanism can be assembled to the housing of the sorting device 1 by any known adapted mechanism, for example by being screwed or riveted to the housing through fixation holes 38.

Apart from this, the sorting device 1 according to the present invention is further provided with a control unit (not visible, advantageously contained in the housing of the device 1) and a driving organ adapted to control the movements of the vessel support 30 with respect to the frame 10. More specifically, the support 30 should be driven for stirring the source vessel 2 with accelerated movements, causing the biological entities contained in the sample to change their positions within the source vessel 2. By adapting the stirring parameters, it is possible to move the biological entities away from the walls of the source vessel 2.

Such a stirring shall not harm the organoids, because the whole liquid or gel medium where they lie is also stirred around with them. Of course, the organoids (more generally, the biological entities as listed above) do move with respect to the liquid or gel, because they have a slightly different density and a large mass which cannot be so easily accelerated by the viscous forces exerted by the surrounding liquid or gel. This remains a much gentler form of mechanical interaction than pushing the organoids around with a solid tip, as may be presently observed in a laboratory, in the absence of any existing alternative solution.

As mentioned earlier, the Applicant noticed that implementation of a stirring in the form of looped trajectories with respect to the frame 10 of the sorting device 1, with adapted predefined shapes and speed parameters, provide relative movements of the plurality of organoids between each other and with respect to the source vessel 2, which may solve the above-mentioned problem.

For reaching that purpose, the driving organ may comprise x-y independent actuators properly controlled by the control unit in such a way that the support 30 might be driven along adapted looped trajectories. Other structures of the driving organ might be implemented without going beyond the scope of the present invention as defined in the appended claims.

For instance, a preferred embodiment for the driving unit is partly illustrated in Fig. 4. According to this specific examplary embodiment, a driving button 40 is eccentrically connected to a driving shaft (not visible) of the driving unit, itself driven in rotation about the z direction by an appropriate electric motor. The driving button 40 comprises a cannon (not visible) connected to the driving shaft and a head of bigger radius than that of the cannon.

The cannon is positioned inside an appropriate circular hole 42, designed in a lateral extension 44 of the support 30 and having a smaller radius than that of the head of the driving button 40, in such a manner that the head can limit the movements of the support 30 along the z direction.

When the driving shaft rotates, the cannon of the driving button 40 is driven along a circle having a bigger radius than that of the hole 42 and, consequently, the driving button 40 drives the support 30 along a looped trajectory in the xy plane, substantially circular or elliptical, in the manner of a crank driving organ.

After carrying out many tests, the Applicant could define preferred parameters for optimizing the effect of the looped trajectories on the entities contained in the samples.

As previously mentioned, a gathering stirring might be implemented in order to move the organoids away from the wall(s) of the source vessel 2.

In any case, the control unit and the driving organ may be arranged so as to provide the vessel support 2 with an acceleration having a radial component of at least 0.1 m/s² ( by "radial component" it is meant, the component of the vessel acceleration which is perpendicular to its instantaneous trajectory often referred to as "centripetal" or "centrifugal" acceleration), while the looped trajectories may preferably have a mean characteristic diameter of 2 cm or smaller, preferably 1 cm or smaller.

In general, the support 30 and the flex mechanism can be made from the same material, possibly as a monolithic structure. But they can also be assembled from independent parts. Possible materials for both the support 30 and the flex structures 34, 36 are plastics or metals. The blades may preferably be metallic (stainless steel, brass, copper) for higher resistance to elastic fatigue. The support 30 may preferably be made of a lower density material, for instance plastic or aluminium. The assembling of the support 30 and the driving unit should ideally involve contact between surfaces made of low-friction materials, for example polyoxymethylene (POM) or polished stainless steel.

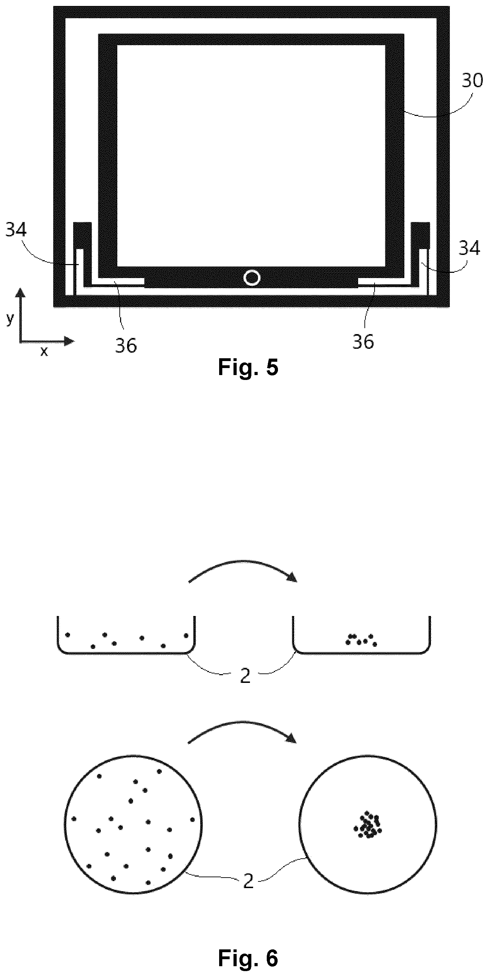

Fig. 5 illustrates an alternative embodiment of a supporting structure for source vessels which is monolithic, i.e. the support and the flex mechanism are made as one piece, and has a slightly different shape than the support 30 illustrated in Fig. 4, in a non-limiting illustrative way.

Additive manufacturing technics might also be implemented to build such components without going beyond the scope of the present invention as defined by the appended claims.

A method according to the present invention, for the selective picking up of at least one biological entity from a source vessel containing a sample including a plurality of biological entities, to be able to selectively dispense this biological entity into a destination vessel, will now be described.

Fig. 6 illustrates, in a lateral view and in a front view, how a preferred kind of stirring operation of a source vessel 2 can be implemented, more precisely a gathering stirring, when at least one biological entity to be selectively picked up is close to a wall of the source vessel 2 (left side of the illustration), so as to be able to substantially gather the plurality of biological entities contained in the corresponding sample inside a central region of the source vessel 2, in the xy plane (right side of the illustration).

On a general basis, the method according to the present invention comprises the steps consisting in:
a) providing a sample including a plurality of biological entities in a source vessel 2 and put the latter down on a vessel support 30 of a sorting device 1 according to the preceding description,
b) assessing the distribution in the source vessel 2 of the plurality of biological entities included in the sample, in particular, if said at least one biological entity to be selectively picked up is close to a wall of the source vessel 2,
c) implementing a stirring operation of the sample, on the basis of a manual request by a user or of an automatic request by the identification and localization system, during which the driving organ drives the vessel support 30 according to successive looped trajectories, inside the xy plane, with predefined shapes and speed parameters which are adapted to stir the sample and provide movements of the plurality of biological entities between each other and with respect to the source vessel 2 so as to be able to substantially gather the plurality of biological entities inside a central region of the source vessel 2, in the xy plane,
d) acquiring at least one image of the sample to locate the at least one biological entity to be selectively picked up, and
e) selectively picking up the at least one biological entity to be selectively picked up.

A user may decide to implement a stirring operation (as well as the kind of stirring operation) on the basis of the result of the assessment carried out in step b), thanks to images acquired by the identification and localization system. Alternatively, it is also possible to provide that the identification and localization system itself is conceived and programmed to carry out an analysis of acquired images (possibly by using an artificial intelligence), before launching then automatically a stirring operation adapted to expected predefined sorting results.

In general, the control unit and the driving organ may be arranged so as to provide the vessel support 30 with an acceleration having a radial component of at least 0.1 m/s².

Centralizing the biological entities or gathering them in a central region of the source vessel 2 is useful when eligible biological entities are lying next to a wall of the source vessel 2. This may be achieved by applying a slow, regular movement along a looped trajectory which might substantially be circular or elliptical. By "slow" it should be understood less than 120 rpm. Considering again the 3 mm radius of the trajectory, this means centrifugal acceleration of less than 0.5 m/s². By "regular" it should be understood that there should be no substantial change in speed or direction. The loop motion might be maintained for at least 10 complete revolutions, typically up to 20, which at 120 rpm represents a duration of the order of 10 s of motion.

Successive stirring operations can also be implemented as a function of the result after every run, which can be assessed by implementing the identification and localization system, without going beyond the scope of the present invention as defined in the appended claims.

Thanks to the above-described features, a sorting device and a sorting method are provided which make it possible to selectively pick up sensitive biological entities from a sample with reduced risks of damages, in a precise and reliable manner.

The one skilled in the art will encounter no particular difficulty to adapt the structure of the support 30, of its assembling to the housing of the device, as well as of the driving organ, as a function of his needs and without going beyond the scope of the present invention as defined by the appended set of claims.

## Claims

1. Sorting device (1) comprising a picking station (4) and an identification and localization system, adapted to allow selective picking up of at least one biological entity from a source vessel (2) containing a sample including a plurality of biological entities, to be able to selectively dispense said at least one biological entity into a destination vessel (6), the sorting device (1) including a frame (10) on which a vessel support (30) for bearing at least one source vessel (2) is mounted in a movable manner, as well as a control unit and a driving organ adapted to control the movements of said vessel support (30) with respect to said frame (10),
**characterized in that** said control unit and said driving organ are arranged so as to drive said vessel support (30) along successive looped trajectories with respect to said frame (10), inside a xy plane, in response to a corresponding manual request by a user or to a corresponding automatic request by said identification and localization system, and
**in that** said control unit and said driving organ are arranged so as to provide said looped trajectories with predefined shapes and speed parameters which are adapted to stir the sample and provide relative movements of the plurality of biological entities between each other and with respect to the source vessel (2) so as to be able to substantially gather the plurality of biological entities inside a central region of the source vessel (2), in the xy plane.

2. Sorting device (1) according to claim 1, **characterized in that** said control unit and said driving organ are arranged so as to provide said vessel support (30) with an acceleration having a radial component of at least 0.1 m/s².

3. Sorting device (1) according to claim 1 or 2, **characterized in that** said control unit and said driving organ are arranged so as to drive said vessel support (30) along successive looped trajectories with a rotation speed of 120 rpm or less, so as to be able to substantially gather the plurality of biological entities inside a central region of the source vessel (2), in the xy plane.

4. Sorting device (1) according to any of the preceding claims, **characterized in that** said looped trajectories are substantially circular or elliptical.

5. Sorting device (1) according to any of the preceding claims, **characterized in that** said looped trajectories have a mean characteristic diameter of 2 cm or smaller, preferably 1 cm or smaller.

6. Sorting device (1) according to any of the preceding claims, **characterized in that** said vessel support (30) is connected to said frame (10) by at least one elastic blade, preferably at least two elastic blades.

7. Sorting device (1) according to any of the preceding claims, **characterized in that** said driving organ comprises a crank arranged in contact with said vessel support (30) to drive the latter along said looped trajectories.

8. Sorting device (1) according to any of the preceding claims,
**characterized in that** said vessel support (30) has at least one see-through window (28), and
**in that** said identification and localization system comprises at least one image acquisition device mounted on said frame (10) and facing said see-through window (28) so as to be able to acquire an image of biological entities included in a sample contained in a transparent source vessel (2) while it is located on said vessel support (30).

9. Method for the selective picking up of at least one biological entity from a source vessel (2) containing a sample including a plurality of biological entities, to be able to selectively dispense said at least one biological entity into a destination vessel (6), **characterized in that** it comprises the steps consisting in:
a) providing a sample including a plurality of biological entities in a source vessel (2) and put the latter down on a vessel support (30) of a sorting device (1) according to any of claims 1 to 8,
b) assessing the distribution in said source vessel (2) of the plurality of biological entities included in said sample, in particular, if said at least one biological entity to be selectively picked up is close to a wall of said source vessel (2),
c) implementing a stirring operation of said sample, on the basis of a manual request by a user or of an automatic request by said identification and localization system, during which said driving organ drives said vessel support (30) according to successive looped trajectories, inside said xy plane, with predefined shapes and speed parameters which are adapted to stir said sample and provide movements of the plurality of biological entities between each other and with respect to said source vessel (2) so as to be able to substantially gather the plurality of biological entities inside a central region of the source vessel (2), in the xy plane,
d) acquiring at least one image of said sample to locate said at least one biological entity to be selectively picked up, and
e) selectively picking up said at least one biological entity to be selectively picked up.

10. Method according to claim 9, **characterized in that** said vessel support (30) is driven by said driving organ with an acceleration having a radial component of at least 0.1 m/s² in step c).

11. Method according to claim 9 or 10, **characterized in that**, if it appears in step b) that said at least one biological entity to be selectively picked up is close to a wall of said source vessel (2), said vessel support (30) is driven, in step c), along successive looped trajectories with a rotation speed of 120 rpm or less, so as to be able to substantially gather the plurality of biological entities inside a central region of said source vessel (2), in said xy plane.

12. Method according to any of claims 9 to 11, **characterized in that** said looped trajectories are substantially circular or elliptical.
